(19) Europäisches Patentamt
European Patent Office
Office européen des brevets

(11) Publication number: 0 329 367 A2

(12) EUROPEAN PATENT APPLICATION

(21) Application number: 89301362.3

(51) Int. Cl.4: C07C 85/24 , C07C 87/50

(22) Date of filing: 14.02.89

(30) Priority: 15.02.88 JP 32550/88
15.02.88 JP 32551/88
08.07.88 JP 170570/88
07.01.89 JP 857/89

(43) Date of publication of application:
23.08.89 Bulletin 89/34

(84) Designated Contracting States:
AT BE CH DE ES FR GB GR IT LI LU NL SE

(71) Applicant: MITSUI PETROCHEMICAL INDUSTRIES, LTD.
2-5, Kasumigaseki 3-chome Chiyoda-ku
Tokyo 100(JP)

(72) Inventor: Kiso, Yoshihisa c/o MITSUI PETROCHEM. IND. LTD 1-2
Waki 6-chome, Waki-cho
Kuga-gun, Yamaguchi(JP)
Inventor: Takai, Toshihiro c/o MITSUI PETROCHEM. IND. LTD 3
Chigusakaigan
Ichihara-shi Chiba(JP)
Inventor: Hayashi, Tetsuo c/o MITSUI PETROCHEM. IND. LTD 1-2
Waki 6-chome, Waki-cho
Kuga-gun Yamaguchi(JP)

(74) Representative: Myerscough, Philip Boyd et al
J.A.Kemp & Co. 14, South Square Gray's Inn
London, WC1R 5EU(GB)

# (54) Method of preparing 4,4'-methylenedianiline.

(57) A process for preparing 4,4'-methylenedianiline which comprises reacting aniline and a methylenating agent in the presence of a catalyst comprising a dealuminized Y type zeolite, a fluorine-treated dealuminized Y type zeolite, or a metal ion-treated product of a dealuminized Y type zeolite having a proton as an exchanged cation.

EP 0 329 367 A2

# METHOD OF PREPARING 4,4′-METHYLENEDIANILINE

## BACKGROUND OF THE INVENTION

### 1. Field of the Invention

The present invention relates to a method of preparing 4,4′-methylenedianiline from aniline and a methylenating agent. More specifically, it relates to a method of preparing 4,4′-methylenedianiline at a high reaction efficiency by using a special catalyst.

### 2. Description of the Related Art

4,4′-methylenedianiline is most useful as the intermediate for production of high purity methylene diisocyanate which is the starting material for polyurethane.

It has been known in the art to prepare 4,4′-methylenedianiline by the reaction between aniline and formaldehyde. In this reaction, the yield differs depending on the catalyst employed, and it has been recognized that the selection of the catalyst is an important technical key to the proper reaction.

As an example, it is known to use a mineral acid such as hydrogen chloride as the catalyst. In this method, a mineral acid as the catalyst must be used in an amount equimolar to aniline or more, and further, an uneconomical disadvantage arises in that an alkali in an amount equimolar to the mineral acid or more is required for neutralization of the mineral acid after the reaction. Moreover, this method also poses a problem in that the yield of 4,4′-methylenedianiline is lowered, because high condensates such as polymethylenepolyphenylamine are formed at an efficiency as high as 20 to 40%.

As a catalyst for alleviating this lowering in the yield of 4,4′-methylenedianiline, a solid acid catalyst has been proposed, and as an example thereof, a Y type zeolite has been proposed (Japanese Patent Publication (Kokoku) No. 55-34138, No. 56-14104 and No. 58-27261). But, in these methods also, in addition to the desired 4,4′-methylenedianiline, 2,4′-methylenedianiline and polymethylene polyphenylamine and the like are formed. The formation of such byproducts cannot be efficiently decreased during the reaction, and therefore, a problem arises in that the yield of 4,4′-methylenedianiline is still low.

## SUMMARY OF THE INVENTION

Accordingly, the objects of the present invention are to solve the above-mentioned problems of the prior art and to provide a method of preparing 4,4′-methylenedianiline from aniline and a methylenating agent selectively at a high yield by inhibiting the formation of high condensates or isomers during the reaction.

Another object of the present invention is to provide a method for preparing 4,4′-methylenedianiline with the same catalyst over a long period, with little deterioration of the catalyst.

Other objects and advantages of the present invention will be apparent from the following description.

In accordance with the present invention, there is provided a method of preparing 4,4′-methylenedianiline comprising the step of reacting aniline and a methylenating agent in the presence, as a catalyst, of a dealuminized Y type zeolite, a fluorine-treated dealuminized Y type zeolite, or a metal ion-treated product of a dealuminized Y type zeolite having a proton as an exchanged cation.

Further, according to the present invention, when a metal ion-treated and fluorine-treated product of a dealuminized Y type zeolite having a proton as the exchanged cation is used as the above catalyst, the activity of the catalyst can be maintained over a long term, and at the same time, the activity of forming 4,4′-methylenedianiline, and the selectivity, is high, whereby an economical effect that a further efficient production of 4,4′-methylenedianiline is rendered possible is obtained.

In accordance with the further preferred embodiment, there is provided a method of preparing 4,4′-methylenedianiline by a dehydration condensation of aniline and a methylenating agent and heating the dehydrated condensate in the presence of a zeolite catalyst, wherein a dealuminized Y type zeolite, a fluorine-treated dealuminized Y type zeolite, or a metal ion-treated product of a dealuminized Y type zeolite having a proton as an exchanged cation is used as the zeolite catalyst, with the water content in the dehydrated condensate being 0.1% by weight or less.

DESCRIPTION OF THE PREFERRED EMBODIMENTS

Methylenating agents to be reacted with aniline according to the present invention include those containing formaldehyde, such as an aqueous formalin solution, trioxane, p-formaldehyde, dialkoxymethane such as dimethoxymethane or the like, and N,N′-diphenylmethylenediamine obtained by mixing aniline and an aqueous formalin solution in the absence of a catalyst. Among the above, as the starting material, N,N′-diphenylmethylenediamine and dialkoxymethane are preferably used, because water is not formed in the reaction, and thus a higher selectivity and activity of 4,4′-methylenedianiline is obtained compared with other starting materials.

The dealuminized Y type zeolite usable as the catalyst in the present invention is obtained by subjecting the Y type zeolite to a dealuminization treatment. The Y type zeolite has a $SiO_2/Al_2O_3$ (molar ratio) of 2 to 5 with a three-dimensional skeletal structure comprising a tetragonal body of $SiO_4$ and $AlO_4$ sharing a mutual oxygen atom, and an electrical balance of the respective tetragonal bodies containing aluminum is maintained by the presence of cations in the aluminosilicate skelton.

The dealuminized Y type zeolite is the product of a dealuminization of a Y type zeolite having a $SiO_2/Al_2O_3$ (mole ratio) of 5.5 to 20 and substantially the same crystal structure as the Y type zeolite, but with the size of a unit lattice generally reduced by about 1 to 2%. The exchanged cation may include a proton, alkaline earth cations, rare earth cations, and transition metal cations, but a proton is preferable.

The Y type zeolite generally has a higher acid strength, heat resistance, acid and base resistance, since the $SiO_2/Al_2O_3$ (mole ratio) is higher, but since a Y type zeolite is not formed with a starting material composition exceeding a $SiO_2/Al_2O_3$ (mole ratio) of 5, in general a Y type zeolite is prepared with a starting material composition having a $SiO_2/Al_2O_3$ (mole ratio) of 2 to 5 and then enhancing the $SiO_2/Al_2O_3$ (mole ratio) while maintaining the basic crystal structure of the Y type zeolite by dealuminization.

As the dealuminized Y type zeolite, commercially available products can be used including, for example, TSZ-330HUA, TSZ-350HUA, TSZ-360HUA (all trade marks) from Toso K.K. Alternately, it can be prepared from a Y type zeolite by the methods (1), (2) as described below.

(1) In the first method, aluminum is eliminated from the skeleton as an Al cation with an EDTA liquid phase. For example, a proton-exchanged type Y type zeolite is added to a solution containing 1 to 10-fold by weight of distilled water and 0.1 to 1-fold by weight of EDTA, and left to stand for about 10 to 200 hours, and then 0.5 to 3-fold by weight of 1 to 3 N HCl is added dropwise, and after heating the mixture while stirring to 100°C, followed by stirring for 1 to 10 hours, the solid phase is separated by filtration or centrifugation, and thereafter, washed with water followed by calcination in air or a nitrogen atmosphere at 300 to 700°C for 1 to 10 hours.

(2) In the second method, a steam treatment is carried out at a high temperature. For example, a proton-exchanged type Y type zeolite in a fixed bed is treated with a steam diluted 10 to 100% with nitrogen at a temperature of 600 to 900°C for 1 to 10 hours, then washed with about 10 to 100-fold by weight of distilled water and calcined in air or a nitrogen atmosphere for 300 to 700°C for 1 to 10 hours. Alternatively, a proton exchanged type Y type zeolite is treated in fluidized bed with steam diluted 10 to 100% with nitrogen at a temperature of 600 to 900°C under a normal pressure or under a pressurized condition for 1 to 10 hours, then washed with about 10 to 100-fold by weight of distilled water, and calcined in air or a nitrogen atmosphere at 300 to 700°C for 1 to 10 hours.

The fluorine-treated dealuminized Y type zeolite usable as the catalyst in the present invention is obtained by treating the dealuminized Y type zeolite as described above with fluorides, to bond the fluorine within the crystal structure, having a $SiO_2/Al_2O_3$ (mole ratio) of 5.5 to 20 and a fluorine content of 0.1 to 30% by weight. The exchange cation may include a proton, alkaline earth cations, rare earth cations, transition metal cations, etc. but a proton is preferable. The proton-exchanged type fluorine-treated dealuminized Y type zeolite has an X-ray diffraction pattern shown in Table A, and has a different structure to those of a dealuminized Y type zeolite and a fluorine-treated Y type zeolite.

Table A

| Diffraction angle (2θ°) | Relative intensity |
|---|---|
| 6.4 ± 0.2 | 90 - 100 |
| 10.4 ± 0.2 | 60 - 80 |
| 12.1 ± 0.2 | 40 - 70 |
| 14.8 ± 0.2 | 1 - 30 |
| 16.0 ± 0.2 | 70 - 90 |
| 19.1 ± 0.2 | 10 - 40 |
| 20.8 ± 0.2 | 20 - 50 |
| 23.3 ± 0.2 | 1 - 20 |
| 24.2 ± 0.2 | 20 - 50 |
| 25.2 ± 0.2 | 1 - 30 |
| 27.6 ± 0.2 | 10 - 30 |
| (Note) X-ray diffraction analysis is conducted by using a CuKα-line, but the relative intensity of the diffraction line of 6.4 ± 0.2 is made 100. | |

The fluorine-treated dealuminized Y type zeolite can be obtained by placing the dealuminized Y type zeolite described above in contact with a fluorine containing compound. As the fluorine containing compound, there can be mentioned ammonium fluoride compounds such as ammonium fluoride, tetramethylammonium fluoride, tetraethylammonium fluoride, methylammonium fluoride, and dimethylammonium fluoride or the like; fluorine containing compounds such as sodium fluoride, hydrogen fluoride, boron trifluoride, monofluoroacetic acid $CFCl_3$ , $CF_2Cl_2$ , $CF_3Cl$, $CF_4$ , $CHFCl_2$ , $CHF_2Cl$, $CHF_3$ , $CFCl_2$-$CFCl_2$ , $CF_2Cl$-$CF_2Cl$, $CF_2Cl$-$CF_3$, $CF_3$-$CF_3$ , $CH_3$-$CF_2Cl$, $CH_3$-$CHF_2$ , $CF_3Br$, $CF_2Br$-$CF_2Br$, HF-$SF_4$ , $SF_6$ , $BF_3$ and others. As the fluoride treatment method, for example, a dealuminized Y type zeolite is dipped in an aqueous solution 0.01 to 30% by weight, preferably 0.1 to 15% by weight, a fluorine containing compound for 0.01 to 24 hours, preferably 0.1 to 5 hours, and the solid phase then separated by filtration or centrifugation and calcined in air or a nitrogen atmosphere at 300 to 800°C, preferably 500 to 750°C. As another method, a dealuminized Y type zeolite is placed in contact with a gaseous fluorine-containing compound at a temperature of 0 to 800°C, preferably 200 to 600°C. More specifically, a dealuminized Y type zeolite is filled in a reaction tube, then after the reaction is set to a predetermined temperature, the above gaseous fluorine containing compound is supplied to the above reaction tube over a predetermined time (for example, 0.1 to 10 hours, preferably 0.5 to 2 hours) to place the dealuminized Y type zeolite in contact with the above gaseous fluorine containing compound, and thereafter, the fluorine containing compound remaining in the reaction tube is removed by replacement with an inert gas such as nitrogen, or a degassing treatment under a reduced pressure.

Another catalyst usable in the present invention is a metal ion-treated product of a dealuminized Y type zeolite having a proton as the exchanged cation (hereinafter called "dealuminized Y type zeolite"), and a catalyst obtained by using the above-mentioned metal ion treatment and the above-mentioned fluorine treatment in combination has specific features that the catalyst activity is greater and the catalyst life is prolonged.

The Y type zeolite may include, as the exchanged cation, a proton, alkaline earth cation, rare earth cation, and transition metal cation. In the present invention, a dealuminized Y type zeolite having particularly a proton as the exchanged cation is selected, is subjected to a metal ion treatment or metal ion treatment and fluorine treatment in combination, and is used as the catalyst. This catalyst has a specific feature in that the 4,4′-dimethylenedianiline formation activity is higher than that of the catalysts having other exchanged cations, and the selectivity thereof is high.

The metal ion treatment of the dealuminized Y-type zeolite may be carried out by dipping the above Y-type zeolite in an aqueous solution of a metal ion compound, as exemplified below, and filtering and drying the product, followed by calcination.

The concentration of the aqueous solution of the metal ion compound may be suitably selected within the range of from 0.01 to 50% by weight. A concentration of 0.1 to 20% by weight is preferred as the ionized concentration. The dealuminized Y type zeolite may be dipped in the aqueous metal ion compound solution at room temperature to 200° C for about 0.1 hour to 20 hours, preferably at 60 to 150° C for 0.2 to 6 hours. Drying after filtration may be performed at room temperature to 200° C for 0.1 to 40 hours, preferably at 60 to 150° C for 0.5 to 20 hours, and calcination, which is the final step, carried out at 100 to 800° C for 0.1 to 40 hours, preferably 500 to 700° C for 0.5 to 10 hours.

The metal of the metal ion may include those shown below.

Alkaline earth metal: Be, Mg, Ca, Sr, Ba, Ra
Group Iva: Ti, Zr, Hf
Group VIIa: Mn, Tc, Re
Group VIII: Fe, Co, Ni, Ru, Rh, Pd, Os, Ir, Pt
Group IIIb: Al, Ga, In, Tl
Group Ivb: Ge, Sn, Pb
Group Vb: Sb, Bi
Lanthanoid elements: La, Ce, Lu, etc.

Among the above, the preferable metal ion compound may be exemplified by Mg, Ca, Sr, Ba, Zr, Mn, Re, Fe, Ru, Rh, Pd, Pt, Al, Sn, Bi, La, Ce, Lu compounds, and particularly preferable metal ion compounds may include Ba, Fe, Ru, Rh, Pt, Bi compounds.

When the dealuminized Y type zeolite is subjected to the metal ion treatment, it is not clear how the structure of the zeolite is changed, but it is thought that the protons in an ordinary zeolite are exchanged with metal ions, and in some cases, that a part of the silica-alumina skeleton is exchanged with metal ions.

As shown in the Reference examples described below, when iron ion is used as the metal ion, the catalyst effect is not satisfactory, with a small amount of content, for exchanging the protons in the zeolite, but desirably the treatment is effected at a content of 5% or higher, and in this case, as a result of an analysis, it was found that the zeolite with iron ions replacing aluminum ions is formed.

When the metal treatment and the fluorine treatment of the dealuminized Y type zeolite are used in combination, which is another embodiment of the present invention, the method in which the fluorine treatment is applied after the metal ion treatment as described above, and the method in which the above fluorine treatment is previously conducted and the metal ion treatment is applied later, may be used. The fluorine treatment after the metal ion treatment is preferable in that the formation activity of 4,4′-methylenedianiline is higher. The fluorine treatment of the dealuminized Y type zeolite can be carried out in the manner mentioned above.

The reactions between aniline and formaldehyde according to the present invention may be represented as follows.

① The method in which aniline and a methylenating agent such as formaldehyde are allowed to react directly with each other in the presence of a catalyst.

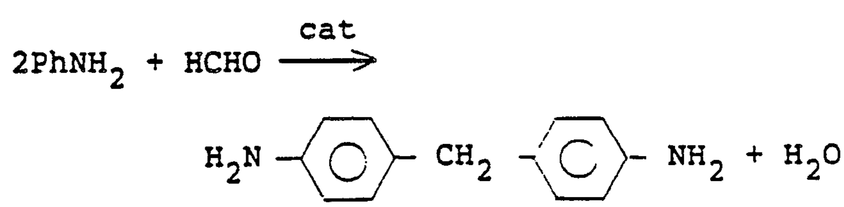


② The method in which aniline and formaldehyde are subjected to dehydration condensation, and water is separated to synthesize intermediates, which are then subjected to the reaction in the presence of a catalyst.

In this case, the dehydrated condensate may include:

$C_6H_5N=CH_2$
$C_6H_5NHCH_2NHC_6H_5$
$C_6H_5NHCH_2NHC_6H_4NH_2$
$C_6H_5NH[CH_2NHC_6H_4NH]_xCH_2C_6H_4NH_2$
(x: 1 or more)

and the substituted positions of the respective substituents on the benzene ring are indefinite.

Next, as the second step, by heating the above dehydrated condensate by using a dealuminized Y type

zeolite, a fluorine-treated dealuminized Y type zeolite, a metal ion-treated product of a dealuminized Y type zeolite having a proton as the exchanged cation, or the dealuminized Y type zeolite by using the metal ion treatment and the fluorine treatment in combination, as the catalyst, the desired 4,4′-methyleneaniline is prepared.

The above method ② is performed at a ratio of aniline/formaldehyde starting material ≧ 3 without isolation of the intermediates. Of the above synthetic methods, the reaction ① has a tendency to lower the activity of the catalyst because of a large amount of water in the reaction system, and therefore, the reaction ② is preferred. The water content in the reaction system is preferably 5000 ppm or less, particularly 1000 ppm or less.

The zeolite catalyst to be used in the present invention also may be used as such, but to ensure an efficient reaction, a catalyst subjected to the heating pre-treatment is preferred. Is the heating pre-treatment method, heating is effected generally at a temperature of 500° C or higher, preferably 650° C or higher, and less than the breaking temperature of the zeolite crystal structure, to dehydrate water in the zeolite.

The catalyst is preferably in a powder or pellet form, and the catalyst concentration is 1 to 200% by weight, preferably 5 to 50% by weight, based on the reaction mixture.

The amounts of aniline and methylenating agent used in the present reaction are preferably 1 to 100, more preferably 2 to 50, most preferably 3 to 20, in terms of the molar ratio of aniline/methylenating agent.

The reaction is preferably conducted in the liquid phase, and in that case, a solvent cannot be employed, but it is also possible to carry out the reaction by using a solvent. Examples of the solvent include aromatic hydrocarbons such as benzene, toluene, and xylene; aliphatic hydrocarbons such as pentane, hexane, heptane, octane, nonane, decane, cyclohexane, and decalin; halogenated hydrocarbons such as dichloromethane, chloroform, and carbon tetrachloride.

According to the first embodiment, preferably the reaction method is used in which, after aniline is placed in contact with the catalyst, the reaction is carried out with an addition of a formaldehyde starting material such as an aqueous formalin solution, and further, the reaction method is used in which aniline and a formaldehyde starting material are mixed in the absence of a catalyst to form a condensate, and after separation of the aqueous phase, the product is placed in contact with a catalyst.

The reaction temperature is preferably 20 to 300° C, more preferably 30 to 180° C, most preferably 50 to 140° C. The pressure may be normal pressure, but pressurization with an inert gas is also possible, to maintain the liquid phase. The reaction time is preferably 0.1 to 40 hours, more preferably 0.3 to 50 hours. The reaction system may be batchwise, semi-batchwise with a liquid phase, or continuous with a fixed bed.

The reaction method according to the second embodiment of the present invention comprises mixing aniline and a methylenating agent in the absence of a catalyst to form a condensate, and separating the aqueous phase. The reaction at this time may be carried out at a temperature of 10 to 200° C, preferably 20 to 100° C, under a normal pressure to 30 kg/cm², preferably a normal pressure to 5 kg/cm², generally for 0.1 to 10 hours, preferably 0.5 to 40 hours. The aqueous phase is separated from the condensate formed by this reaction by a reduced pressure aspiration, such as a rotary evaporator, or other general hydration methods such as dehydrating instruments, to obtain a crude dehydrated condensate. Further, the crude dehydrated condensate can be dehydrated by a high purity drying method using a drying agent such as a molecular sieve 3 Å, or by removing water by distillation by introducing an azeotropic agent for water such as benzene, whereby a dehydrated condensate having a reduced water content of 0.1% by weight or less, preferably 0.05% by weight or less, can be obtained.

The dehydrated condensate includes:

$C_6H_5N=CH_2$

$C_6H_5NHCH_2NHC_6H_5$

$C_6H_5NHCH_2NHC_6H_4NH_2$

$C_6H_5NH[CH_2NHC_6H_4NH]_xCH_2C_6H_4NH_2$

(x: 1 or more)

and the substituted positions of the respective substituents on the benzene ring are indefinite.

Next, as the second step, the dehydrated condensate obtained as described above is heated in the presence of a zeolite catalyst to produce the desired 4,4′-methylenedianiline.

The reaction method in the second step of the present invention comprises adding the zeolite catalyst to the dehydrated condensate having a water content of 0.1% by weight or less, and carrying out the reaction at a temperature of 20 to 300° C, preferably 30 to 180° C, more preferably 40 to 140° C, under a pressure of a normal pressure to 30 kg/cm², preferably a normal pressure to 5 kg/cm², for a time of 0.1 to 20 hours, preferably 0.2 to 5 hours, whereby 4,4′ dimethyldianiline can be prepared.

In this reaction, preferably the aniline added in excess in the first step coexists in the reaction system.

The reaction system according to the preparation method as described above may be batchwise, semi-batchwise with a liquid phase, or continuous with a fixed bed.

According to the present invention, by using the above-specified dealuminized Y type zeolite as the catalyst, the formation of a high condensate and isomers can be suppressed to selectively produce 4,4′-methylenediamine at high yield, and further, the catalyst is little deactivated and thus can be used repeatedly. Particularly, when the catalyst subjected to a combination of the metal ion treatment and the fluorine treatment is used, the catalyst activity can be maintained over a long term, whereby the number of catalyst exchanges can be reduced, which brings an economical advantage.

According to another embodiment of the present invention, by carrying out the reaction with a dehydrated condensate having a water content of 0.1% by weight or less, and by using a zeolite catalyst having a good reaction efficiency, the selectivity of the desired product, 4,4′-methylenedianiline, can be improved, and further, the reaction can be improved compared with the prior art.

## Examples

The present invention will now be further illustrated by, but is by no means limited to, the following Examples.

## Example 1

A mixture of 400.4 g of anilin and 69.8 g of aqueous 37% by weight formalin was stirred at a normal temperature for 5 hours, the mixture was left to stand overnight, and the aqueous layer was separated and dehydrated in a rotary evaporator at 50 °C under a reduced pressure of 300 mmHg for 4 hours to obtain a N,N′-diphenylmethylenediamine/aniline mixture. The structure was confirmed by $^{13}C$ NMR, and it was found that 100% of the formaldehyde had reacted and been converted to N,N′-diphenylmethylenediamine.

Next, 2.5 g of the N,N′-diphenylmethylenediamine/aniline mixture and 0.50 g of the proton exchanged type dealuminized Y type zeolite TSZ-330HOA (trade mark, manufactured by Toso K.K., $SiO_2/Al_2O_3$ (molar ratio) = 5.5 to 6.5) were added to a 25 ml four-necked flask equipped with a reflux condenser, under $N_2$ at room temperature, and the reaction was carried out at 120 °C for 3 hours. After cooling to room temperature, the reaction product was poured into a mixed system of 100 ml of diethyl ether and 100 ml of an aqueous 10% by weight sodium hydroxide solution, and the organic layer was analyzed by GC and GPC.

The results are shown in Table 1.

## Examples 2 - 4

Example 1 was repeated, except that the catalyst or the reaction conditions were changed.

The results are shown in Table 1.

## Comparative Example 1

Example 1 was repeated, except that the proton exchanged type Y type zeolite (manufactured by Toso K.K., trade mark: TSZ-320HOA) was used. The results are shown in Table 1.

From the above results, it can be seen that the products of the Examples have a higher 4,4′-methylenedianiline selectivity (yield) than the product of the Comparative Example.

## Example 5

Example 1 was repeated, except 2.34 g of aniline and 0.40 g of an aqueous 37% by weight formalin solution was used, instead of the N,N′-diphenylmethylenediamine/aniline mixture, as the starting material.

The results are shown in Table 1.

Table 1

| | Catalyst | Reaction temperature (°C) | Aniline conversion (mole %) | Selectivity (mole %) | | |
|---|---|---|---|---|---|---|
| | | | | 4,4'-Methylene-dianiline | 2,4'-Methylene-dianiline | Polymethylene Polyphenyl-amine |
| Example 1 | Proton exchange type dealuminized Y type zeolite TSZ-330HUA | 120 | 37 | 84 | 10 | 5 |
| " 2 | " TSZ-330HUA | 90 | 38 | 84 | 5 | 8 |
| " 3 | " TSZ-350HUA | 120 | 38 | 82 | 11 | 6 |
| " 4 | " TSZ-360HUA | 120 | 38 | 79 | 13 | 7 |
| Comp. Ex. 1 | Proton exchanged type Y type zeolite TSZ-320HOA | 120 | 37 | 77 | 12 | 10 |
| Example 5 | Proton exchanged type dealuminized Y type zeolite TSZ-330HUA | 120 | 36 | 79 | 12 | 8 |

From the above results, it can be seen that the products of the Examples gave a higher aniline conversion and 4,4′-methylenediamine selectivity (yield) than that of the Comparative Example.

Example 6

A catalyst of a dealuminated Y type zeolite having a proton as the exchanged cation, which was subjected to the metal ion treatment, was prepared as described below.

As the dealuminized Y type zeolite having a proton as the exchanged cation of the starting material, TSZ-330 manufactured by Toso K.K. (Si 33.7% by weight, Al 8.67% by weight, hereinafter abbreviated as HUSY) was used.

A quantity of 3.0 g of HUSY zeolite was added to 30 ml of an aqueous 1.0 N $Fe_2(SO_4)_3$ solution, the mixture was heated and stirred at 100°C, the product was separated by filtration and washed twice with 100 ml of distilled water, and after drying at 100°C for 12 hours, the dried product was calcined at 600°C for 3 hours. From elemental analysis of the catalyst, it was found that 9.45% by weight of Fe was attached to the zeolite.

On the other hand, 35.5 g (0.44 mole) of 37% aqueous formalin was added dropwise under ice-cooling to 201.1 g (2.16 mole) of aniline, and the mixture was stirred under room temperature for 3 hours. After standing overnight, the separated aqueous layer was removed, and water was removed by an evaporator (50°C, 20 mmHg) followed by $N_2$ bubbling to obtain 204 g of a dehydrated mixture. An analysis of the mixture revealed that it was a mixture of aniline and $C_6H_5NHCH_2NHC_6H_5$ with a substantially 100% conversion of formaldehyde.

Next, 2.50 g of the above mixture (25 mmol as the aniline unit) and 0.50 g of the above catalyst subjected to the metal treatment were placed in an ampoule replaced with $N_2$ , and the mixture was heated and stirred at 90°C for 30 minutes. After the reaction, 30 ml of an aqueous 5% caustic soda solution was added, and after stirring, the mixture was transferred into a separation funnel.

Further, 70 ml of an aqueous 5% caustic soda solution and 100 ml of diethyl ether were added, the mixture was well shaken to extract the reaction product into the ether layer. The ether solution was analyzed by GPC and GC, and it was found that the aniline conversion was 39%, with a yield of 4,4′-methyldianiline (hereinafter abbreviated as 4,4′-MDA) of 34.6%. The selectivity of the product based on converted aniline was as shown in Table 2.

Examples 7 to 27

Example 6 was repeated, except that the metal salt used in preparation of the catalyst was changed as shown in Table 2.

The results are shown in Table 2.

Table 2

| Example | Catalyst (metal wt%) | Metal salt used during catalyst preparation | Aniline conversion % | Yield of 4,4'-MDA % | Selectivity (%) | | | |
|---|---|---|---|---|---|---|---|---|
| | | | | | 4,4'-MDA | 2,4'-MDA | Intermediate | PMPPA* |
| 6 | $Fe^{3+}$-HUSY ( 9.45) | $Fe_2(SO_4)_3$ | 39 | 34.6 | 88 | 4 | 4 | 4 |
| 7 | $Fe^{3+}$-HUSY (15.7 ) | $FeCl_3$ | 39 | 34.4 | 87 | 4 | 4 | 5 |
| 8 | $Fe^{3+}$-HUSY (16.92) | $Fe(NO_3)_3$ | 39 | 33.5 | 85 | 4 | 5 | 6 |
| 9 | $Ba^{2+}$-HUSY ( 2.84) | $Ba(NO_3)_2$ | 38 | 32.5 | 86 | 4 | 4 | 6 |
| 10 | $Mg^{2+}$-HUSY ( 0.50) | $Mg(NO_3)_2$ | 38 | 31.9 | 84 | 5 | 5 | 7 |
| 11 | $Ca^{2+}$-HUSY ( 0.95) | $Ca(NO_3)_2$ | 39 | 32.0 | 83 | 5 | 5 | 7 |
| 12 | $Sr^{2+}$-HUSY ( 2.44) | $Sr(NO_3)_2$ | 38 | 31.5 | 82 | 5 | 5 | 8 |
| 13 | $La^{3+}$-HUSY ( 2.94) | $La(NO_3)_3$ | 38 | 31.4 | 82 | 5 | 5 | 8 |
| 14 | $Ce^{3+}$-HUSY ( 2.66) | $Ce(NO_3)_3$ | 38 | 30.8 | 82 | 5 | 5 | 8 |
| 15 | $Lu^{3+}$-HUSY ( 3.47) | $Lu(NO_3)_3$ | 39 | 32.8 | 85 | 5 | 5 | 6 |
| 16 | $Zr^{4+}$-HUSY (13.70) | $Zr(NO_3)_4$ | 39 | 31.3 | 80 | 5 | 5 | 9 |
| 17 | $Mn^{2+}$-HUSY ( 1.40) | $Mn(NO_3)_2$ | 39 | 29.5 | 77 | 23 | | |
| 18 | $Re^{7+}$-HUSY ( 0.17) | $NH_4ReO_4$ | 39 | 30.7 | 79 | 6 | 6 | 10 |

Table 2 (Continued)

| Example | Catalyst (metal wt%) | Metal salt used during catalyst preparation | Aniline conversion % | Yield of 4,4'-MDA % | Selectivity (%) | | | |
|---|---|---|---|---|---|---|---|---|
| | | | | | 4,4'-MDA | 2,4'-MDA | Intermediate | PMPPA* |
| 19 | $Ru^{3+}$-HUSY ( 0.20) | $RuCl_3$ | 38 | 33.8 | 88 | 4 | 4 | 5 |
| 20 | $Rh^{3+}$-HUSY ( 1.75) | $Rh(NO_3)_3$ | 38 | 32.8 | 87 | 4 | 4 | 5 |
| 21 | $Rd^{2+}$-HUSY (32.2 ) | $Pd(NO_3)_2$ | 39 | 32.5 | 83 | 5 | 5 | 8 |
| 22 | $Pt^{4+}$-HUSY ( 0.18) | $H_2PtCl_6$ | 39 | 33.5 | 87 | 4 | 4 | 5 |
| 23 | $Al^{3+}$-HUSY ( 7.03) | $Al(NO_3)_3$ | 38 | 30.4 | 79 | 6 | 5 | 9 |
| 24 | $In^{3+}$-HUSY (20.75) | $In(NO_3)_3$ | 39 | 29.4 | 76 | 7 | 6 | 11 |
| 25 | $Sn^{2+}$-HUSY (13.14) | $SnCl_2$ | 39 | 32.4 | 82 | 5 | 5 | 8 |
| 26 | $Sn^{4+}$-HUSY (18.8 ) | $SnCl_4$ | 39 | 32.9 | 84 | 5 | 5 | 7 |
| 27 | $Bi^{3+}$-HUSY (40.94) | $Bi(NO_3)_3$ | 39 | 34.3 | 88 | 4 | 3 | 5 |

* PMPPA = Polymethylene polyphenylamine

Reference Example 1

Method of treatment of dealuminized Y type zeolite having aluminum in components exchanged with iron ions:

As the dealuminized Y type zeolite having a proton as the exchanged cation of the starting material, HUSY zeolite used in Example 6 was employed.

A quantity of 3.0 g of HUSY zeolite was added to an aqueous $Fe(NO_3)_3$ with a concentration shown in Table 2, the mixture was heated and stirred at 100°C for 3 hours, the product was separated by filtration and washed twice with 100 ml of distilled water, and then the product was dried at 100°C for 12 hours, followed by calcination at 600°C for 3 hours.

The results of elemental analysis of the catalyst thus obtained are shown in Table 3.

Table 3

| Aqueous $FE(NO_3)_3$ treatment of proton exchanged type dealuminized Y type zeolite | | | | |
|---|---|---|---|---|
| No. | Aqueous $Fe(NO_2)_3$ conc. N | Analysis of zeolite | | |
| | | Si | Al | Fe |
| 1 | 2.0 N | 29.4 | 0.48 | 16.66 |
| 2 | 1.0 N | - | - | 16.92 |
| 3 | 0.25N | - | - | 7.20 |
| 4 | 0.10N | 28.4 | 5.28 | 1.69 |
| 5 | untreated | 33.7 | 8.07 | 0 |

The catalyst No. 2 was used as the catalyst in Example 8 ($Fe^{3+}$-HUSY).

Examples 28 to 30

The same reaction as in Example 6 was carried out for the catalysts obtained by No. 1, 3 and 4. The results are shown in Table 4.

Table 4

| Example | Catalyst | Aniline Conversion (%) | 4,4'-MDA yield (%) | Selectivity | | | |
|---|---|---|---|---|---|---|---|
| | | | | 4,4'-MDA | 2,4'-MDA | Intermediate | PMPPA |
| 28 | No. 1 in Table 3 | 38 | 32.9 | 86 | 4 | 5 | 6 |
| 29 | No. 3 in Table 3 | 39 | 30.8 | 80 | 5 | 5 | 9 |
| 30 | No. 4 in Table 3 | 38 | 26.1 | 73 | | 27 | |

Example 31

Using $Ba(NO_3)_2$, an HUSY zeolite was treated the same method as in Example 6. A quantity of 2.5 g of the $Ba^{2+}$-HUSY zeolite was added to 50 ml of an aqueous 1.0 by weight $NH_4F$ solution, and the reaction was carried out under stirring at 50° C for one hour, followed by air cooling and separation by filtration. The product was dried at 100° C for 12 hours, and then calcined at 600° C for 3 hours. As the result of elemental analysis, the Ba was 3.23% by weight, F 0.79% by weight, Si 28.6% by weight, and Al 7.17% by weight. When the reaction was carried out in the same manner as in Example 6 by using this catalyst, the aniline conversion was 39% and the 4,4'-MDA yield 33.2%. The selectivities to 4,4'-MDA, 2,4'-MDA,

intermediate, and PMPPA were 86, 4, 4, and 6%, respectively.

Reference Example 2 (catalyst preparation):

A 5.0 g amount of a proton exchanged type dealuminized Y type zeolite TSZ-330HUS (trade mark, manufactured by Toso K.K., $SiO_2/Al_2O_3$ (molar ratio) = 5.5 to 6.5) was added to 100 ml of a 2.5% by weight aqueous ammonium fluoride solution, and after the mixture was stirred at 50 °C for one hour, the solid was filtered dried at 100 °C for 12 hours, and further, was calcined at 600 °C for 3 hours to obtain a fluorine-treated dealuminized Y type zeolite.

The physical properties and X-ray analyses of this product were as shown in Tables 5 and 6.

Reference Example 3 (catalyst preparation):

Reference Example 2 was repeated, except that the concentration of the aqueous ammonium fluoride solution was changed to 1.9% by weight to obtain a fluorine-treated dealuminized Y type zeolite.

The physical properties of this product were as shown in Table 5.

Table 5

| | Ref. Ex. 2 | Ref. Ex. 3 | Dealuminzed Y type zeolite |
|---|---|---|---|
| F content (wt.%) | 11.0 | 5.5 | - |
| $SiO_2/Al_2O_3$ (mole ratio) | 8.2 | 8.1 | 7.5 |
| Specific surface area ($m^2/g$) | 562 | 599 | 686 |
| Acid content* (meq/g) | 0.95 | 1.23 | 3.48 |
| Crystal structure | Table 6 | - | Table 7 |

*: Determined by an ammonia temperature elimination elevation method (after absorption of ammonia at 100 °C, ammonia is eliminated and quantitatively determined by elevating the temperature to 700 °C)

Table 6

| X-ray diffraction of proton exchanged type fluorine-treated dealuminized Y type zeolite | |
|---|---|
| Diffraction angle (2θ°) | Relative intensity |
| 6.4 | 100 |
| 10.4 | 70 |
| 12.1 | 55 |
| 14.8 | 15 |
| 16.0 | 80 |
| 19.1 | 25 |
| 20.8 | 35 |
| 23.3 | 5 |
| 24.2 | 35 |
| 25.2 | 10 |
| 26.4 | 5 |
| 27.6 | 20 |

Table 7

| X-ray diffraction of proton exchanged type dealuminized Y type zeolite | |
|---|---|
| Diffraction angle (2θ°) | Relative intensity |
| 6.3 | 100 |
| 10.3 | 70 |
| 12.1 | 60 |
| 15.9 | 90 |
| 19.0 | 30 |
| 20.7 | 40 |
| 23.2 | 10 |
| 24.0 | 55 |
| 26.2 | 5 |
| 27.5 | 30 |

Example 22

To a 25 ml four-necked flask equipped with a reflux condenser were added 2.5 g of the N,N′-diphenylmethylenediamine/aniline mixture obtained in the same manner as in Example 1 and 0.5 g of the fluorine-treated dealuminized Y type zeolite of Reference Example 3, under $N_2$ at room temperature, and the reaction was carried out at 120°C for 30 minutes. After cooling to room temperature, the supernatant was withdrawn to be separated from the catalyst, and a part of the supernatant was analyzed by GC and GPC.

To the separated catalyst was added 2.5 g of N,N′-diphenylmethylenediamine/aniline mixture, and the same operations as described above were repeated three times.

Comparative Example 2

Example 32 was repeated, except that the proton exchanged type dealuminized Y type zeolite before fluorine-treatment was used.

The results are shown in Table 8.

Table 8

(mole %)

| Repetition time | | Example 32 | Comp. Ex. 2 |
|---|---|---|---|
| 1 | Aniline conversion | 40 | 39 |
| | 4,4′-Methylenedianiline selectivity | 80 | 79 |
| | 2,4′-Methylenedianiline selectivity | 10 | 10 |
| | 4-Aminobenzylaniline selectivity | 1.7 | 2 |
| | Polymethylene polyphenylamine selectivity | 8 | 9 |
| 2 | Aniline conversion | 39 | 38 |
| | 4,4′-Methylenedianiline selectivity | 77 | 72 |
| | 2,4′-Methylenedianiline selectivity | 9 | 8 |
| | 4-Aminobenzylaniline selectivity | 2.5 | 5 |
| | Polymethylene polyphenylamine selectivity | 11 | 15 |
| 3 | Aniline conversion | 39 | 39 |
| | 4,4′-Methylenedianiline selectivity | 76 | 52 |
| | 2,4′-Methylenedianiline selectivity | 9 | 9 |
| | 4-Aminobenzylaniline selectivity | 3.5 | 9 |
| | Polymethylene polyphenylamine selectivity | 12 | 30 |
| 4 | Aniline conversion | 39 | 37 |
| | 4,4′-Methylenedianiline selectivity | 76 | 32 |
| | 2,4′-Methylenedianiline selectivity | 8 | 17 |
| | 4-Aminobenzylaniline selectivity | 3.7 | 21 |
| | Polymethylene polyphenylamine selectivity | 13 | 31 |

From the above results it can be seen that the products of the Examples gave a higher aniline conversion and 4,4′-methylenedianiline selectivity compared with that of the Comparative Example, and further, there was little deactivation of the catalyst, thus enabling a repeated usage thereof.

Example 33

The reaction was carried out between 2.5 g of the N,N′-diphenylmethylenediamine/aniline mixture prepared in the same manner as in Example 32 and 0.5 g of the fluorine-treated dealuminized Y type zeolite of Reference Example 3 in the same manner as the first time in Example 32 at 120°C for 3 hours. The results of the reaction are shown in Table 9.

Example 34

Example 33 was repeated, except that the zeolite of Reference Example 2 was used.

The results of the reaction are shown in Table 9.

Table 9

| | (mole %) | |
|---|---|---|
| | Example 33 | Example 34 |
| Aniline conversion | 38 | 37 |
| 4,4′-Methylenedianiline selectivity | 82 | 82 |
| 2,4′-Methylenedianiline selectivity | 12 | 12 |
| 4-Aminobenzylaniline selectivity | 0.8 | 0.7 |
| Polymethylene polyphenylamine selectivity | 5 | 5 |

Example 35

The dehydrated condensate having a water content of 0.18% by weight and obtained in the same manner as in Example 1 was dehydrated with a molecular sieve 3 Å to a water content of 0.05% by weight.

Next, 2.5 g of the N,N′-diphenylmethylenediamine/aniline mixture and 0.50 g of the proton exchanged type dealuminized Y type zeolite TSZ-330HUA (trade mark, manufactured by Toso K.K., $SiO_2/Al_2O_3$ (molar ratio) = 5.5 to 6.5) subjected to the heating pretreatment at 600 °C for 3 hours were added to a 25 ml flask equipped with a reflux condenser, and the reaction was carried out at 120 °C for 3 hours. After cooling to room temperature, the reaction product was poured into a mixed system of 100 ml of diethyl ether and 100 ml of an aqueous 10% by weight sodium hydroxide solution, and the organic layer was analyzed by GC and GPC. The results are shown in Table 10.

Examples 36 - 40

Example 35 was repeated, except that the catalyst, the pretreatment conditions, the reaction temperature, and the reaction time were changed as shown in Table 10.

The results are shown in Table 10.

Table 10

| No. | Catalyst | | Water content of dehydrated condensate (wt%) | Reaction temp. (°C) | Reaction time (hr) | Aniline conversion (mole %) | Selectivity (mole %) | | | |
|---|---|---|---|---|---|---|---|---|---|---|
| | | Pretreatment condition | | | | | 4,4'-Methylenedianiline | 2,4' Methylenedianiline | PMPPA | Intermediate |
| Example 35 | TSZ-330HUA | 600°C 3 hrs | 0.05 | 120 | 3 | 39 | 84 | 10 | 5 | <1 |
| " 36 | TSZ-330HUA | 700°C 6 hrs | 0.05 | 120 | 3 | 39 | 85 | 9 | 4 | <1 |
| " 37 | TSZ-330HUA | 700°C 6 hrs | 0.05 | 90 | 3 | 38 | 88 | 6 | 5 | 1 |
| " 38 | TSZ-330HUA | 700°C 6 hrs | 0.05 | 60 | 24 | 40 | 91 | 3 | 4 | 2 |
| " 39 | TSZ-350HUA | 700°C 6 hrs | 0.05 | 90 | 3 | 39 | 90 | 5 | 4 | <1 |
| " 40 | TSZ-350HUA | 700°C 6 hrs | 0.05 | 60 | 24 | 41 | 92 | 3 | 4 | 2 |

## Claims

1. A process for preparing 4,4′-methylenedianiline which comprises reacting aniline and a methylenating agent in the presence of a catalyst comprising a dealuminized Y type zeolite, a fluorine-treated dealuminized Y type zeolite, or a metal ion-treated product of a dealuminized Y type zeolite having a proton as an exchanged cation.

2. A process according to Claim 1, wherein the methylenating agent comprises an aqueous formalin solution, trioxane, or N,N′-diphenylmethylenediamine.

3. A process according to Claim 1 or 2 wherein the dealuminized Y type zeolite has a $SiO_2$ to $Al_2O_3$ mole ratio of from 5.5:1 to 20:1.

4. A process according to Claim 1 or 2, wherein the fluorine-treated dealuminized Y type zeolite has a $SiO_2$ to $Al_2O_3$ mole ratio of from 5.5:1 to 20:1 and a fluorine content of from 0.1% to 30% by weight.

5. A process according to Claim 1, 2 or 4, wherein the catalyst comprises a metal ion-treated and fluorine-treated product of a dealuminized Y type zeolite having a proton as an exchanged cation.

6. A process according to any of Claims 1,2,4 or 5, wherein the metal ion with which the dealuminized Y type zeolite is treated is an ion of Mg, Ca, Sr, Ba, Zr, Mn, Re, Fe, Ru, Rh, Pd, Pt, Al, Sn, Bi, La, Ce or Lu.

7. A process according to any one of the preceding claims wherein the molar ratio of aniline to methylenating agent is from 1:1 to 100:1.

8. A process according to any one of the preceding claims wherein the catalyst is in powder or pellet form and is used in an amount of from 1 to 200% by weight based on the reaction mixture.

9. A process for preparing 4,4′-methylenedianiline which comprises mixing aniline and a methylenating agent in the absence of a catalyst to form a condensate, separating the aqueous phase, and heating the dehydrated condensate in the presence of a zeolite catalyst.

10. A process according to Claim 9 in which the water content of the dehydrated condensate is 0.1% or less.